# EUROPEAN PATENT SPECIFICATION

(11) **EP 4 041 331 B1**
(45) Date of publication and mention of the grant of the patent: **20.05.2026**
(21) Application number: 20788738.1
(22) Date of filing: 05.10.2020
(51) Int. Cl.: A61M 1/00

(54) **NEGATIVE PRESSURE WOUND THERAPY SYSTEMS AND METHODS WITH MULTIPLE NEGATIVE PRESSURE SOURCES**
UNTERDRUCK-WUNDTHERAPIESYSTEME UND -VERFAHREN MIT MEHREREN UNTERDRUCKQUELLEN
SYSTÈMES ET PROCÉDÉS DE THÉRAPIE DE PLAIES PAR PRESSION NÉGATIVE À MULTIPLES SOURCES DE PRESSION NÉGATIVE

(30) Priority: 07.10.2019 GB 201914427
(43) Date of publication of application: 17.08.2022
(73) Proprietor: T.J. Smith and Nephew, Limited, Hull HU3 2BN (GB)
(72) Inventor: ELDER, David Michael, Hull HU3 2BN (GB)
(74) Representative: Appleyard Lees IP LLP
(86) International application number: PCT/EP2020/077851
(87) International publication number: WO 2021/069377

(56) References cited:
- WO-A1-2012/057881
- WO-A1-2016/103031
- WO-A1-2017/191149
- US-A1- 2014 343 518
- US-A1- 2018 140 466
- US-A1- 2018 140 467

## Description

### TECHNICAL FIELD

The present disclosure relates to apparatuses, systems, and methods (not claimed) for the treatment of wounds, for example using dressings in combination with negative pressure wound therapy.

### DESCRIPTION OF THE RELATED ART

The treatment of open or chronic wounds that are too large to spontaneously close or otherwise fail to heal by means of applying negative pressure to the site of the wound is well known in the art. Negative pressure wound therapy (NPWT) systems currently known in the art commonly involve placing a cover that is impermeable or semi- permeable to fluids over the wound, using various means to seal the cover to the tissue of the patient surrounding the wound, and connecting a source of negative pressure (such as a vacuum pump) to the cover in a manner so that negative pressure is created and maintained under the cover. It is believed that such negative pressures promote wound healing by facilitating the formation of granulation tissue at the wound site and assisting the body's normal inflammatory process while simultaneously removing excess fluid, which may contain adverse cytokines and/or bacteria. However, further improvements in NPWT are needed to fully realize the benefits of treatment.
US 2014/343518 A1 discloses a wound care device for treating wounds by means of subatmospheric pressure. US 2018/140466 A1 and US 2018/140467 A1 disclose devices and methods for treating a wound of a patient with negative pressure.
Further relevant prior art is for instance disclosed in documents WO2016/103031 A1, WO2017/191149 A1 and WO2012/057881 A1.

### SUMMARY

A negative pressure wound therapy system according to the present invention comprises the technical features as defined in independent claim 1.

The present disclosure is related to a negative pressure wound therapy system that includes a wound dressing, a first source of negative pressure disposed on or within the wound dressing, a second source of negative pressure disposed on or within the wound dressing, and electronic circuitry disposed on or within the wound dressing. The wound dressing can be configured to be placed over a wound of a patient and the wound dressing can be configured to absorb fluid. The second source of negative pressure can be pneumatically connected in series with the first source of negative pressure. The electronic circuitry can be configured to generate a first driving signal with a first driving signal magnitude and a first driving signal frequency. The electronic circuitry can be configured to apply the first driving signal to the first and second sources of negative pressure. The electronic circuitry can be configured to cause the first and
second sources of negative pressure to provide negative pressure to aspirate fluid from the wound.

The system of the preceding paragraph and/or any of the systems disclosed herein may include any combination of the following features described in this paragraph, among other features described herein. In some cases, applying the first driving signal to the first and second sources of negative pressure pneumatically connected in series can cause provision of negative pressure to the wound at a maximum negative pressure level that is greater than individual maximum negative pressure levels of the first and second sources of negative pressure. The maximum negative pressure level can be equal to (or possibly greater than) a combined individual maximum negative pressure level of the first and second sources of negative pressure. The first source of negative pressure can include a first piezoelectric transducer. The second source of negative pressure can include a second piezoelectric transducer. The first driving signal frequency can correspond to a resonant frequency of at least one of the first or second piezoelectric transducers. The electronic circuitry can be further configured to: determine the resonant frequency at initialization of the system, monitor current resonant frequency during operation of the system, and in response to a determination that the current resonant frequency is different from the resonant frequency determined at the initialization of the system, set the resonant frequency to the current resonant frequency.

The system of any of the preceding paragraphs and/or any of the systems disclosed herein may include any combination of the following features described in this paragraph, among other features described herein. The first source of negative pressure can include a first piezoelectric transducer. The second source of negative pressure can include a second piezoelectric transducer. The first driving signal frequency can be different from a resonant frequency of at least one of the first or second piezoelectric transducers. The first piezoelectric transducer can have a first resonant frequency and the second piezoelectric transducer can have a second resonant frequency that is different from the first resonant frequency. The electronic circuitry can be configured to: generate a second driving signal with a second driving signal magnitude and a second driving signal frequency, apply the first driving signal to the first piezoelectric transducer of the first source of negative pressure, and apply the second driving signal to the second piezoelectric transducer of the second source of negative pressure, the first driving signal frequency substantially corresponding to the first resonant frequency and the second driving signal frequency substantially corresponding to the second resonant frequency.

In some cases, a method (not claimed) for operating a negative pressure wound therapy system is performed by electronic circuitry of the negative pressure wound therapy system disposed in or on a wound dressing of the negative pressure wound therapy system. The method can include generating a first pumping signal with a first pumping signal magnitude and a first pumping signal frequency. The method can include applying the first pumping signal to a first source of negative pressure of the negative pressure wound therapy system. The method can include applying the first pumping signal to a second source of negative pressure of the negative pressure wound therapy system. The first source of negative pressure can be disposed on or within the dressing. The second source of negative pressure can be disposed on or within the dressing. The first source of negative pressure and the second source of negative pressure can be pneumatically connected in series with each other. In some cases, applying the first pumping signal to the first and second sources of negative pressure can cause the first and second sources of negative pressure to collectively provide negative pressure at a maximum negative pressure level that is greater than individual maximum negative pressure levels of the first and second sources of negative pressure.

The method of any of the preceding paragraphs and/or any of the methods disclosed herein may include any combination of the following features or steps described in this paragraph, among other features described herein. The maximum negative pressure level can be equal to or greater than combined maximum negative pressure levels of the first and second sources of negative pressure. The first source of negative pressure can include a first piezoelectric transducer. The second source of negative pressure can include a second piezoelectric transducer. The first pumping signal frequency can correspond to a resonant frequency of at least one of the first or second piezoelectric transducers. The method can further include: determining the resonant frequency at initialization of the system, monitoring current resonant frequency during operation of the system, and in response to determining that the current resonant frequency is different from the resonant frequency determined at the initialization of the system, updating the resonant frequency to correspond to the current resonant frequency.

The method of any of the preceding paragraphs and/or any of the methods disclosed herein may include any combination of the following features or steps described in this paragraph, among other features described herein. The first source of negative pressure can include a first piezoelectric transducer. The second source of negative pressure can include a second piezoelectric transducer. The first pumping signal frequency can be different from a resonant frequency of at least one of the first or second piezoelectric transducers. The first piezoelectric transducer can be associated with a first resonant frequency. The second piezoelectric transducer can be associated with a second resonant frequency that is different from the first resonant frequency. The method can further include: generating a second pumping signal with a second pumping signal magnitude and a second pumping signal frequency, applying the first pumping signal to the first piezoelectric transducer of the first source of negative pressure, and applying the second pumping signal to the second piezoelectric transducer of the second source of negative pressure, the first pumping signal frequency substantially corresponding to the first resonant frequency and the second pumping signal frequency substantially corresponding to the second resonant frequency.

In some cases, a negative pressure wound therapy system includes a first source of negative pressure, a second source of negative pressure, and electronic circuitry. The first source of negative pressure can be configured to supply negative pressure to a wound covered by a wound dressing. The second source of negative pressure can be configured to supply negative pressure to the wound covered by the wound dressing. The second source of negative pressure can be pneumatically connected in series with the first source of negative pressure. The electronic circuitry can be configured to generate a first driving signal with a first driving signal frequency. The electronic circuitry can be configured to apply the first driving signal to the first and second sources of negative pressure. The electronic circuitry can be configured to cause the first and second sources of negative pressure to provide negative pressure collectively at a maximum negative pressure level that is greater than individual maximum negative pressure levels of the first and second sources of negative pressure.

The system of any of the preceding paragraphs and/or any of the systems disclosed herein may include any combination of the following features described in this paragraph, among other features described herein. The system can further include a housing enclosing the first and second sources of negative pressure and the electronic circuitry. The maximum negative pressure level can be equal to (or possibly greater than) a combined individual maximum negative pressure level of the first and second sources of negative pressure. The first source of negative pressure can include a first piezoelectric transducer and the second source of negative pressure comprises a second piezoelectric transducer. The first driving signal frequency can correspond to a resonant frequency of at least one of the first or second piezoelectric transducers.

The system of any of the preceding paragraphs and/or any of the systems disclosed herein may include any combination of the following features described in this paragraph, among other features described herein. The first source of negative pressure can include a first piezoelectric transducer and the second source of negative pressure comprises a second piezoelectric transducer. The first driving signal frequency can be different from a resonant frequency of at least one of the first or second piezoelectric transducers. The electronic circuitry can be further configured to determine the resonant frequency at initialization of the system, monitor current resonant frequency during operation of the system, and in response to a determination that the current resonant frequency is different from the resonant frequency determined at the initialization of the system, set the resonant frequency to the current resonant frequency. The first piezoelectric transducer can be associated with a first resonant frequency. The second piezoelectric transducer can be associated with a second resonant frequency different from the first resonant frequency. The electronic circuitry can be further configured to generate a second driving signal with a second driving signal frequency, apply the first driving signal to the first piezoelectric transducer of the first source of negative pressure, and apply the second driving signal to the second piezoelectric transducer of the second source of negative pressure, the first driving signal frequency substantially corresponding to the first resonant frequency and the second driving signal frequency substantially corresponding to the second resonant frequency.

Any of the features of any of the methods described herein can be used with any of the features of any of the other methods described herein. Any of the features of any of the systems, devices, or methods illustrated in the figures or described herein can be used with any of the features of any of the other systems, devices, or methods illustrated in the figures or described herein.

### BRIEF DESCRIPTION OF THE DRAWINGS

FIGS. 1A-1C illustrate a wound dressing incorporating a source of negative pressure and/or other electronic components within the wound dressing;
FIGS. 2A-2B illustrate an electronics unit that may be incorporated into a wound dressing;
FIG. 3 is an exploded perspective view of an electronics assembly enclosing an electronics unit within a housing;
FIG. 4A illustrates a bottom perspective view of the electronics assembly of FIG. 3;
FIG. 4B illustrates a top perspective view of the electronics assembly of FIG. 3;
FIG. 5A is an exploded view of a wound dressing incorporating an electronics assembly within the wound dressing layers;
FIG. 5B illustrates a cross sectional layout of the material layers of a wound dressing incorporating an electronics assembly within the dressing;
FIGS. 6A-6B and 7A-7B illustrate components of an electronics assembly;
FIGS 8A-8C illustrate a reduced pressure wound therapy device;
FIG. 9 illustrates an example NPWT system that includes multiple pumps; and
FIG. 10 illustrates a process for operating a NPWT system.

### DETAILED DESCRIPTION

The present disclosure relates to apparatuses and methods (not claimed) of treating a wound with reduced pressure, including a source of negative pressure and wound dressing components and apparatuses. These apparatuses and components, including but not limited to wound overlays, backing layers, cover layers, drapes, sealing layers, spacer layers, absorbent layers, transmission layers, wound contact layers, packing materials, fillers and/or fluidic connectors are sometimes collectively referred to herein as dressings.

It will be appreciated that throughout this specification reference is made to a wound. It is to be understood that the term wound is to be broadly construed and encompasses open and closed wounds in which skin may be torn, cut or punctured or where trauma causes a contusion, or any other superficial or other conditions or imperfections on the skin of a patient or otherwise that benefit from reduced pressure treatment. A wound is thus broadly defined as any damaged region of tissue where fluid may or may not be produced. Examples of such wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, stoma, surgical wounds, trauma and venous ulcers or the like.

It will be understood that embodiments of the present disclosure are generally applicable to use in NPWT or topical negative pressure ("TNP") therapy systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping stabilize the tissue in the apposed position of closure. A further beneficial use of TNP therapy can be found in grafts and flaps where removal of excess fluid is important and close proximity of the graft to tissue is required in order to ensure tissue viability.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, 1013.25 mbar, etc.) (1 mmHg = 133.322 pascals (Pa)). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (such as,-40 mmHg is less than -60 mmHg). Negative pressure that is "more" or "greater" than -X mmHg corresponds to pressure that is further from atmospheric pressure (such as, -80 mmHg is more than -60 mmHg). In some cases, local ambient atmospheric pressure is used as a reference point, and such local atmospheric pressure may not necessarily be, for example, 760 mmHg.

The negative pressure range can be approximately -80 mmHg, or between about -20 mmHg and -200 mmHg. Note that these pressures are relative to normal ambient atmospheric pressure, which can be 760 mmHg. Thus, -200 mmHg would be about 560 mmHg in practical terms. In some cases, the pressure range can be between about -40 mmHg and -150 mmHg. Alternatively, a pressure range of up to -75 mmHg, up to -80 mmHg or over -80 mmHg can be used. Also in some cases a pressure range of below -75 mmHg can be used. Alternatively, a pressure range of over approximately -100 mmHg, or even -150 mmHg, can be supplied by the negative pressure apparatus.

### Wound Dressing

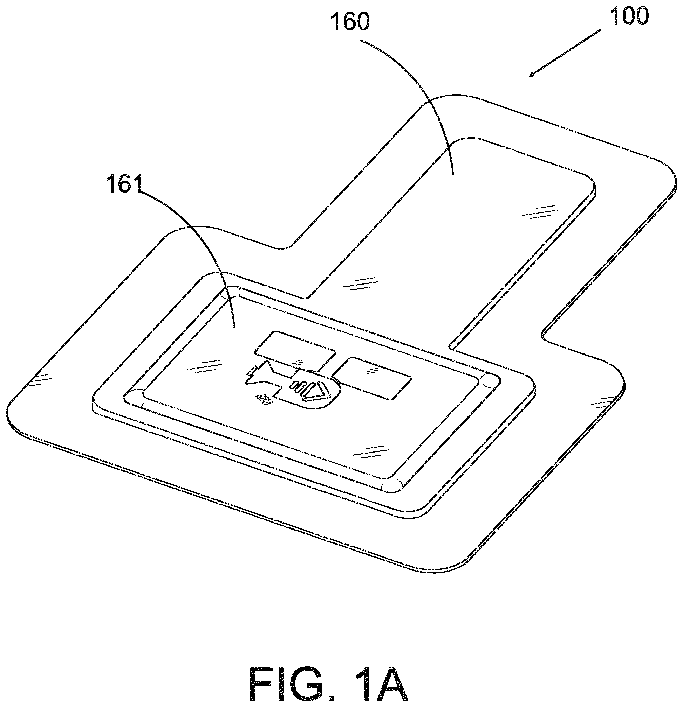

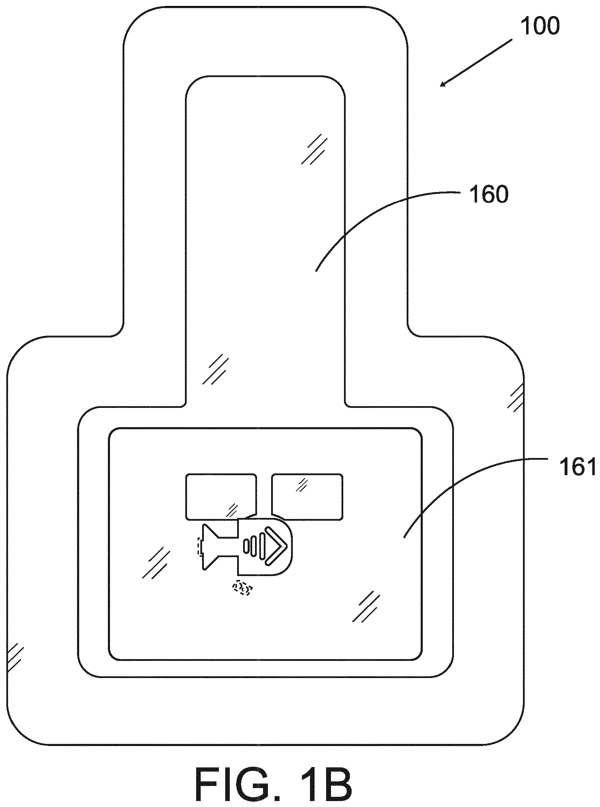

A source of negative pressure (such as a pump) and some or all other components of the TNP system, such as power source(s), sensor(s), connector(s), user interface component(s) (such as button(s), switch(es), speaker(s), screen(s), etc.) and the like, can be integral with the wound dressing. The material layers can include a wound contact layer, one or more absorbent layers, one or more transmission or spacer layers, and a backing layer or cover layer covering the one or more absorbent and transmission or spacer layers. The wound dressing can be placed over a wound and sealed to the wound with the pump and/or other electronic components contained under the cover layer within the wound dressing. The dressing can be provided as a single article with all wound dressing elements (including the pump) pre-attached and integrated into a single unit. A periphery of the wound contact layer can be attached to the periphery of the cover layer enclosing all wound dressing elements as illustrated in FIGS. 1A-1C.

The pump and/or other electronic components can be configured to be positioned adjacent to or next to the absorbent and/or transmission layers so that the pump and/or other electronic components are still part of a single article to be applied to a patient. The pump and/or other electronics can be positioned away from the wound site. Although certain features disclosed herein may be described as relating to systems and method for controlling operation of a negative pressure wound therapy system in which the pump and/or other electronic components are positioned in or on the wound dressing, the systems and methods disclosed herein are applicable to any negative pressure wound therapy system or any medical device. FIGS. 1A-1C illustrate a wound dressing incorporating the source of negative pressure and/or other electronic components within the wound dressing. FIGS. 1A-1C illustrate a wound dressing 100 with the pump and/or other electronics positioned away from the wound site. The wound dressing can include an electronics area 161 and an absorbent area 160. The dressing can comprise a wound contact layer 110 (not shown in FIGS. 1A-1B) and a moisture vapor permeable film, cover layer or backing layer 113 positioned above the contact layer and other layers of the dressing. The wound dressing layers and components of the electronics area as well as the absorbent area can be covered by one continuous cover layer 113 as shown in FIGS. 1A-1C.

A layer 111 of porous material can be located above the wound contact layer 110. As used herein, the terms porous material, spacer, and/or transmission layer can be used interchangeably to refer to the layer of material in the dressing configured to distribute negative pressure throughout the wound area. This porous layer, or transmission layer, 111 allows transmission of fluid including liquid and gas away from a wound site into upper layers of the wound dressing. In particular, the transmission layer 111 preferably ensures that an open air channel can be maintained to communicate negative pressure over the wound area even when the absorbent layer has absorbed substantial amounts of exudates. The layer 111 should preferably remain open under the typical pressures that will be applied during negative pressure wound therapy as described above, so that the whole wound site sees an equalized negative pressure. The layer 111 may be formed of a material having a three dimensional structure. For example, a knitted or woven spacer fabric (for example Baltex 7970 weft knitted polyester) or a non-woven fabric could be used.

Further, one or more absorbent layers (such as layers 122, 151) for absorbing and retaining exudate aspirated from the wound can be utilized. A superabsorbent material can be used in the absorbent layers 122, 151. The one or more layers 122, 151 of absorbent material may be provided above the transmission layer 111. Since in use each of the absorbent layers experiences negative pressures, the material of the absorbent layer can be chosen to absorb liquid under such circumstances. The absorbent layers 122. 151 may comprise a composite comprising superabsorbent powder, fibrous material such as cellulose, and bonding fibers. The composite can be an airlaid, thermally-bonded composite.

The electronics area 161 can include a source of negative pressure (such as a pump) and some or all other components of the TNP system, such as power source(s), sensor(s), connector(s), user interface component(s) (such as button(s), switch(es), speaker(s), screen(s), etc.) and the like, that can be integral with the wound dressing. For example, the electronics area 161 can include a button or switch 114 as shown in FIGS. 1A-1B. The button or switch 114 can be used for operating the pump (such as, turning the pump on/off).

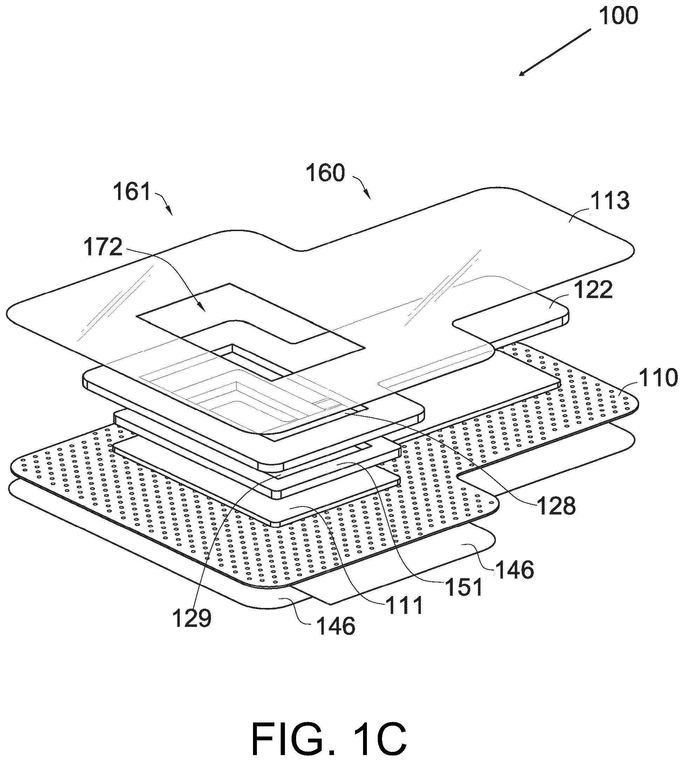

The electronics area 161 of the dressing can comprise one or more layers of transmission or spacer material and/or absorbent material and electronic components can be embedded within the one or more layers of transmission or spacer material and/or absorbent material. The layers of transmission or absorbent material can have recesses or cut outs to embed the electronic components within whilst providing structure to prevent collapse. As shown in FIG. 1C, recesses 128 and 129 can be provided in absorbent layers 151 and 122, respectively.

As used herein the upper layer, top layer, or layer above refers to a layer furthest from the surface of the skin or wound while the dressing is in use and positioned over the wound. Accordingly, the lower surface, lower layer, bottom layer, or layer below refers to the layer that is closest to the surface of the skin or wound while the dressing is in use and positioned over the wound. Additionally, the layers can have a proximal wound-facing face referring to a side or face of the layer closest to the skin or wound and a distal face referring to a side or face of the layer furthest from the skin or wound.

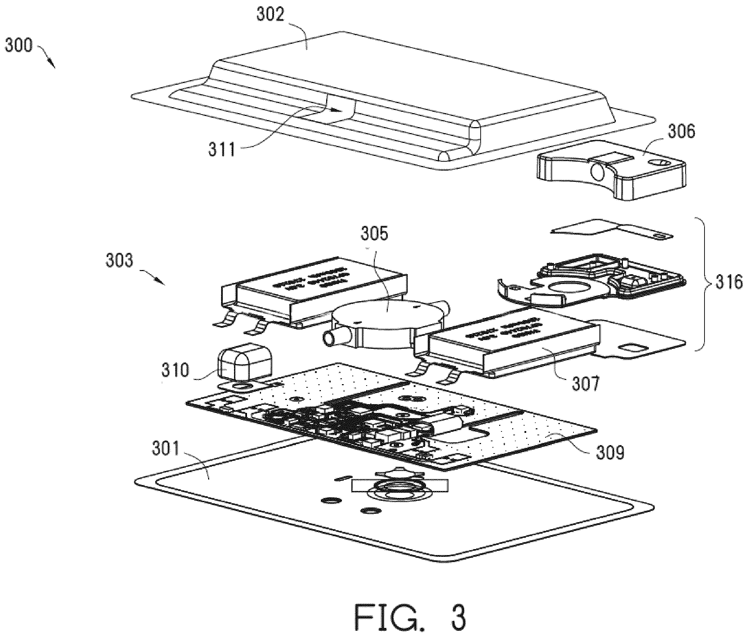

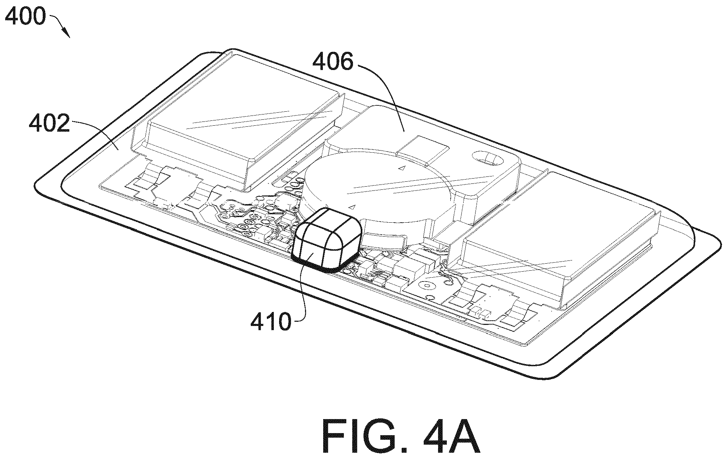

The cover layer may include a cutout 172 positioned over at least a portion of the aperture 128 in the absorbent layer 122 to allow access and fluid communication to at least a portion of the absorbent layers 122 and 151, transmission layer 111, and would contact layer 110 positioned below. An electronics assembly such as described below can be positioned in the apertures 128, 129, and 172 of the first and second absorbent material 151 and 122 and the cover layer 113. The electronics assembly can include a pump, power source, and a printed circuit board as described with reference to FIGS. 3 and 4A-4B.

Before use, the dressing can include one or more delivery layers 146 adhered to the bottom surface of the wound contact layer. The delivery layer 146 can cover adhesive or apertures on the bottom surface of the wound contact layer 110. The delivery layer 146 can provided support for the dressing and can assist in sterile and appropriate placement of the dressing over the wound and skin of the patient. The delivery layer 146 can include handles that can be used by the user to separate the delivery layer 146 from the wound contact layer 110 before applying the dressing to a wound and skin of a patient.

### Electronics Assembly Incorporated Within the Wound Dressing

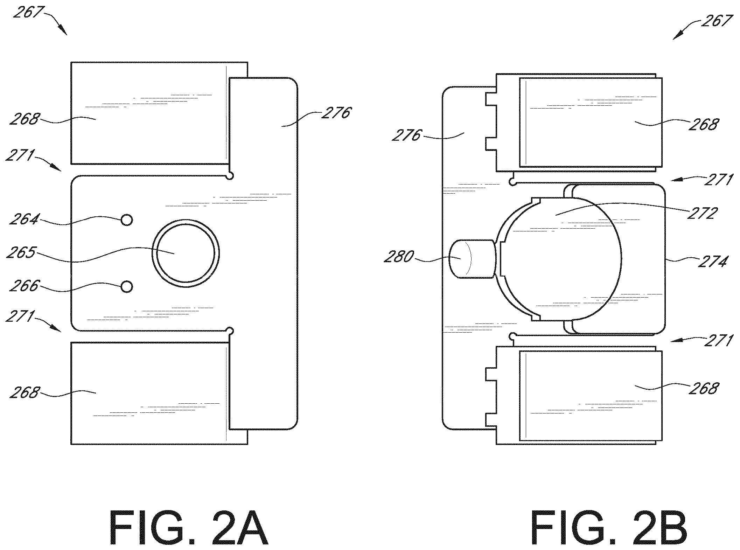

FIGS. 2A-2B illustrate an electronics unit 267 that can be incorporated into a wound dressing. FIG. 2A illustrates the top view of the electronics unit. FIG. 2B illustrates a bottom or wound facing surface of the electronics unit. The electronics unit 267 can include a pump 272 and one or more power sources 268, such as batteries. The electronics unit 267 can include a circuit board 276 configured to be in electrical communication with the pump 272 and/or power source 268. The circuit board 276 can be flexible or substantially flexible.

As illustrated in FIG. 2A, the electronics unit 267 can include single button or switch 265 on the upper surface of the unit. The single button or switch 265 can be used as an on/off button or switch to stop and start operation of the pump and/or electronic components. The electronics unit 267 can also include one or more vents or exhaust apertures 264 on the circuit board 276 for expelling the air exhausted from the pump. As shown in FIG. 2B, a pump outlet exhaust mechanism 274 (sometimes referred to as pump exhaust mechanism or pump outlet mechanism) can be attached to the outlet of the pump 272.

The electronics unit 267 can include a pump inlet protection mechanism 280 as shown in FIG. 2B positioned on the portion of the electronics unit closest to the absorbent area and aligned with the inlet of the pump 272. The pump inlet protection mechanism 280 is positioned between the pump inlet and the absorbent area or absorbent layer of the dressing. The pump inlet protection mechanism 280 can include hydrophobic material to prevent fluid from entering the pump 272. The pump inlet protection mechanism 280 (or any of the inlet protection mechanisms disclosed herein) can include a filter.

The upper surface of the electronics unit 267 can include one or more indicators 266 for indicating a condition of the pump and/or level of pressure within the dressing. The indicators can be small LED lights or other light source that are visible through the dressing components or through holes in the dressing components above the indicators. The indicators can be green, yellow, red, orange, or any other color. For example, there can be two lights, one green light and one orange light. The green light can indicate the device is working properly and the orange light can indicate that there is some issue with the pump (such as, leak, saturation level of the dressing, blockage downstream of the pump, exhaust blockage, low battery, or the like).

The power source 268 can be in electrical communication with the circuit board 276. One or more power source connections are connected to a surface of the circuit board 276. The circuit board 276 can have other electronics incorporated within. For example, the circuit board 276 may support various sensors including, but not limited to, one or more pressure sensors, temperature sensors, optic sensors and/or cameras, and/or saturation indicators.

FIG. 3 illustrates an electronics assembly 300 enclosing an electronics unit within a housing. As illustrated in FIG. 3, the housing of the electronics assembly 300 can include a plate 301 and flexible film 302 enclosing the electronics unit 303 within. The electronics unit 303 can include a pump 305, inlet protection mechanism 310, pump exhaust mechanism 306, power source 307, and circuit board 309. The circuit board 309 can be flexible or substantially flexible.

As is illustrated, the pump exhaust mechanism 306 can be an enclosure, such as a chamber. The electronics unit 303 and pump 305 can be used without the inlet protection mechanism 310. However, the pump exhaust mechanism 306 and the pump 305 can sit within an extended casing 316.

The flexible film 302 can be attached to the plate 301 to form a fluid tight seal and enclosure around the electronic components. The flexible film 302 can be attached to the plate at a perimeter of the plate by heat welding, adhesive bonding, ultrasonic welding, RF welding, or any other attachment or bonding technique.

The flexible film 302 can include an aperture 311. The aperture 311 can allow the inlet protection mechanism 310 to be in fluid communication with the absorbent and/or transmission layers of the wound dressing. The perimeter of the aperture 311 of the flexible film 303 can be sealed or attached to the inlet protection mechanism 310 to form a fluid tight seal and enclosure around the inlet protection mechanism 310 allowing the electronic components 303 to remain protected from fluid within the dressing. The flexible film 302 can be attached to the inlet protection mechanism 310 at a perimeter of the inlet protection mechanism 310 by heat welding, adhesive bonding, ultrasonic welding, RF welding, or any other attachment or bonding technique. The inlet protection mechanism 310 can prevent wound exudate or liquids from the wound and collected in the absorbent area 660 of the wound dressing from entering the pump and/or electronic components of the electronics assembly 300.

The electronics assembly 300 illustrated in FIG. 3 can be incorporated within the wound dressing such that, once the dressing is applied to the body of the patient, air from within the dressing can pass through the inlet protection mechanism 310 to be pumped out toward the pump exhaust mechanism 306 in communication with an aperture in the casing 316 and the circuit board 309 as described herein.

4A-4B illustrate an electronics assembly 400 including a pump inlet protection mechanism 410 sealed to the exterior of the flexible film 402, similar to the description with reference to FIG. 3. Also shown is an exhaust mechanism 406, which can be similar to the exhaust mechanism 306.

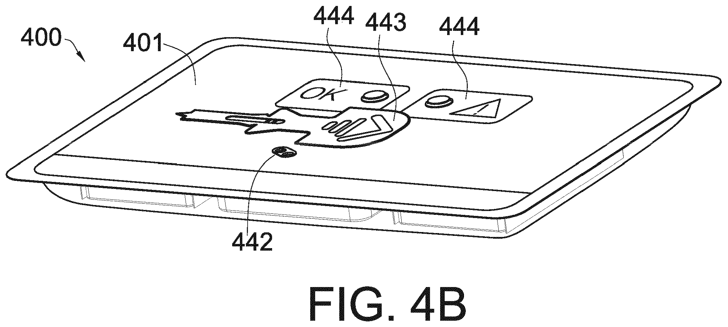

FIG. 4A illustrates lower, wound facing surface of the electronics assembly 400. FIG. 4B shows an upper surface of the plate 401 (which can face the patient or user) of the electronics assembly 400. The upper surface of the plate 401 can include an on/off switch or button cover 443, indicators 444, and/or one or more vent holes 442.

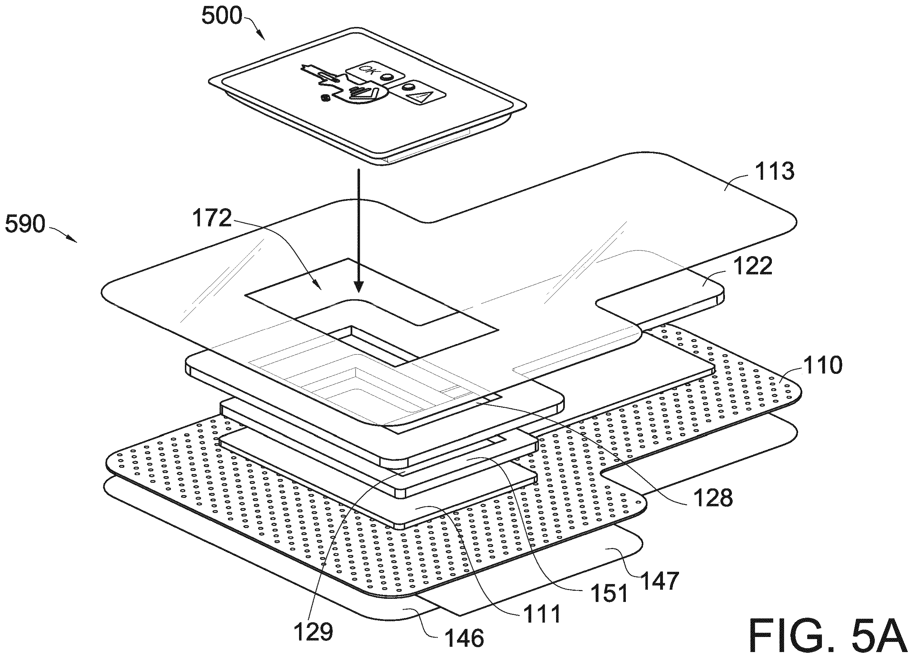

The electronics assembly 400 with the pump inlet protection mechanism 410 extending from and sealed to the film 402 can be positioned within the aperture 172 in the cover layer 113 and absorbent layer(s) (122, 151) as shown in FIG. 1C. The perimeter of the electronics assembly 400 can be sealed to a top surface of the outer perimeter of the aperture 172 in the cover layer 113 as shown in FIGS. 1C and described in more detail with reference to FIG. 5A-5B herein. The electronics assembly 400 can be sealed to the cover layer 113 with a sealant gasket, adhesive, heat welding, adhesive bonding, ultrasonic welding, RF welding, or any other attachment or bonding technique. The electronics assembly 400 can be permanently sealed to the cover layer 113 and could not be removed from the cover layer without destroying the dressing.

The electronics assembly 400 can be utilized in a single dressing and disposed of with the dressing. In some cases, the electronics assembly 400 can be utilized in a series of dressings.

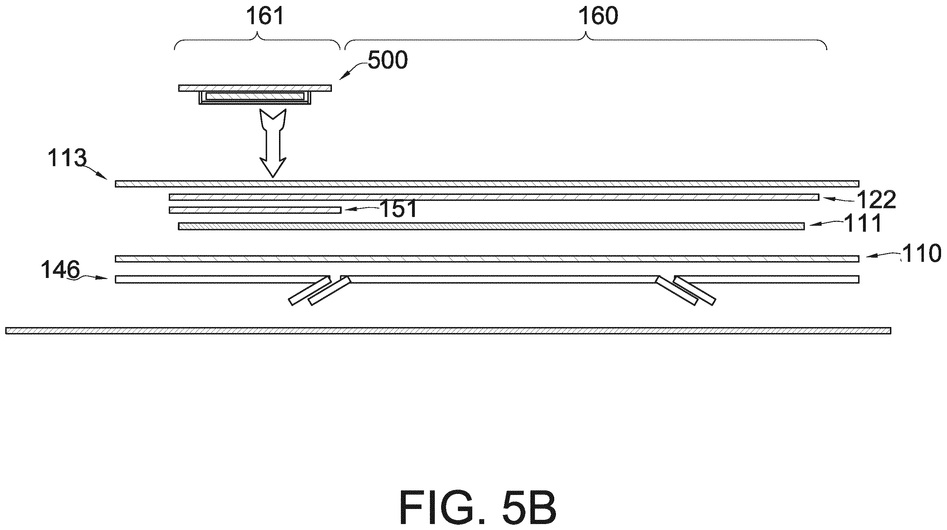

FIG. 5A illustrates a wound dressing, such as the one in FIG. 1C, incorporating an electronics assembly 500 within the wound dressing layers 590. FIG. 5B illustrates a cross-sectional view of the wound dressing incorporating the electronics assembly of FIG. 5A. The electronics assembly 500 can be provided within the aperture 172 in the cover layer and apertures 129 and 128 in the first and second absorbent layers 122, 151. The electronics assembly 500 can seal to the outer perimeter of the aperture 172 of the cover layer. The dressing can comprise a wound contact layer 110 and a moisture vapor permeable film, cover layer or backing layer 113 positioned above the contact layer 110 and other layers of the dressing. A layer 111 of porous material can be located above the wound contact layer 110. As used herein, the terms porous material, spacer, and/or transmission layer can be used interchangeably to refer to the layer of material in the dressing configured to distribute negative pressure throughout the wound area. This porous layer, or transmission layer, 111 allows transmission of fluid including liquid and gas away from a wound site into upper layers of the wound dressing. Further, one or more absorbent layers (such as layers 122, 151) for absorbing and retaining exudate aspirated from the wound can be utilized. The one or more layers 122, 151 of absorbent material may be provided above the transmission layer 111. There may be a small apertured absorbent layer 151 and a large aperture absorbent layer 122. The small apertured absorbent layer 151 can be positioned on top of the large apertured absorbent layer 122. In some cases, the small apertured absorbent layer 151 can be positioned below of the large apertured absorbent layer 122. Before use, the dressing can include one or more delivery layers 146 adhered to the bottom surface of the wound contact layer. The delivery layer 146 can cover adhesive or apertures on the bottom surface of the wound contact layer 110.

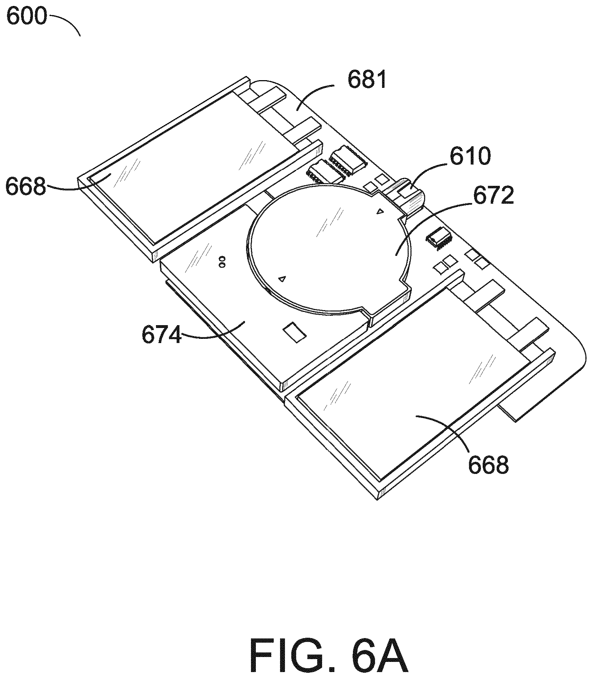

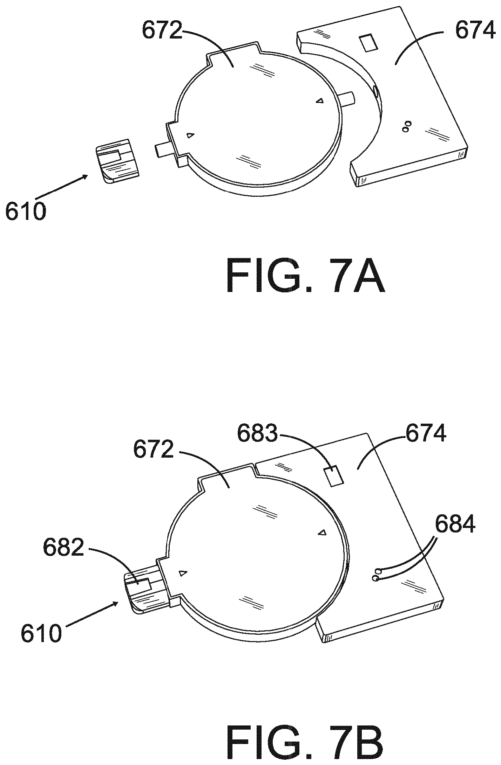

FIGS. 6A-6B and 7A-7B illustrate an electronics assembly 600 with a pump inlet protection mechanism 610 and pump exhaust mechanism 674 on a pump 672. The assembly 600 can include cavities 682 and 683 (shown in FIGS. 7A-7B) on the pump inlet protection mechanism 610 and pump exhaust mechanism 674, respectively. The inlet protection and pump exhaust mechanisms can be adhered to the inlet and the outlet of the pump as described herein. The assembly 600 can be assembled using an adhesive and allowed to cure prior to incorporating into the electronics assembly.

The pump inlet can be covered or fitted with a pump inlet protection mechanism 610. The pump inlet protection 610 can be pushed onto the pump inlet as illustrated by the arrows in FIG. 7A. This can be a friction fit. The port of the pump inlet protection 610 that receives a portion of the pump inlet can be sized and shaped to be a complementary fit around the pump inlet. The pump inlet protection 610 can be bonded onto the pump inlet using a silicone sealant or any other sealant or sealing technique. FIG. 7B illustrates the pump inlet protection mechanism 610 covering the pump inlet and the pump exhaust mechanism 674 covering the pump outlet. The pump exhaust mechanism 674 can include one or more apertures or vents 684 to allow gas aspirated by the pump to be exhausted from the pump exhaust mechanism 674. In some cases, a non-return valve and/or filter membrane of the pump exhaust mechanism is included in the pump exhaust mechanism 674.

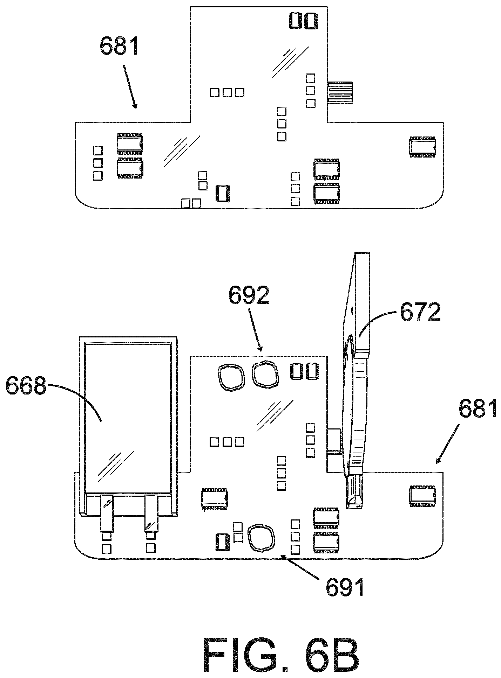

FIGS. 7A-7B illustrate the pump inlet protection mechanism 610 and pump exhaust mechanism 674 with cavities 682 and 683. A pump assembly including the pump inlet protection mechanism 610 and pump exhaust mechanism 674 can be placed over the surface of a circuit board 681. When the pump assembly is in contact with the surface of the circuit board 681, the cavities 682 and 683 can at least partially enclose sensors on the circuit board 681, for example, pressure sensors 691 and 692 on the circuit board 681, as illustrated in FIG. 6B.

The pressure sensors 691 and 692 illustrated in FIG. 6B can be used to measure and/or monitor the pressure level at the wound and atmospheric pressure. The pressure sensor 691 can be used to measure and/or monitor pressure at the wound (such as, underneath the wound dressing), which can be accomplished by measuring and/or monitoring pressure in a fluid flow path connecting the negative pressure source or pump 672 and the wound. The pressure sensor 691 can measure and/or monitor pressure in the cavity 682 of the pump inlet protection mechanism 610 shown in FIGS. 7A-7B.

The pressure sensor 692 can be used to measure and/or monitor pressure external to the wound dressing. The pressure sensor 692 can measure and/or monitor pressure in the cavity 683 of the pump exhaust mechanism 674 shown in FIGS. 7A-7B. The pressure sensor 692 can measure pressure external to the wound dressing, which can be relative atmospheric pressure since the atmospheric pressure varies depending on, for instance, an altitude of use or pressurized environment in which the TNP apparatus may be used. These measurements can be used to establish a desired negative pressure differential (or target negative pressure or setpoint) at the wound relative to the external pressure.

The circuit board 681 (including any of the circuit boards described herein) can include control circuitry, such as one or more processors or controllers, that can control the supply of negative pressure by the negative pressure source 672 according at least to a comparison between the pressure monitored by the pressure sensor 691 and the pressure monitored by the pressure sensor 692. Control circuity can operate the negative pressure source 672 in a first mode (that can be referred to as an initial pump down mode) in which the negative pressure source 672 is activated to establish the negative pressure setpoint at the wound. The setpoint can be set to, for example, a value in the range between about -70 mmHg to about -90 mmHg, among others. Once the setpoint has been established, which can be verified based on a difference between pressure measured by the pressure sensor 691 (or wound pressure) and pressure measured by the pressure sensor 692 (or external pressure), control circuitry can deactivate (or pause) operation of the negative pressure source 672. Control circuitry can operate the negative pressure source 672 is a second mode (that can be referred to as maintenance pump down mode) in which the negative pressure source 672 is periodically activated to re-establish the negative pressure setpoint when the wound is depressurized as a result of one or more leaks in the fluid flow path, which may be caused, among other things, by an imperfect seal between the dressing and skin or tissue surrounding the wound. Control circuitry can activate the negative pressure source 672 in response to the pressure at the wound (as monitored by the pressure sensor 691) becomes more positive than a negative pressure threshold, which can be set to the same negative pressure as the setpoint or lower negative pressure.

Any of the wound dressings, wound treatment apparatuses and methods described herein may also be used in combination or in addition to one or more features described in PCT International Application No. PCT/EP2017/060464, filed May 3, 2017, titled NEGATIVE PRESSURE WOUND THERAPY DEVICE ACTIVATION AND CONTROL, U.S. Patent No. 8,734,425, and U.S. Patent No. 8,905,985.

One or more self-adhesive gaskets can be applied to the pump inlet protection mechanism 610 and pump exhaust mechanism 674 to seal the cavities 682 and 683 of the pump inlet and pump exhaust around sensors on the circuit board 681 and to seal around the exhaust mechanism vent(s) and corresponding vent(s) in the circuit board 681 (as described herein). A pre-formed adhesive sheet can be used to form the sealing gaskets between the cavities 682 and 683 of the pump inlet and pump exhaust mechanisms and sensors on the circuit board 681 and between the exhaust mechanism vent(s) and vent(s) in the circuit board 681. An adhesive can be used to seal the cavities 682 and 683 of the pump inlet protection 610 and pump exhaust mechanism 674 around sensors on the circuit board 681 and to seal around the exhaust mechanism vent(s) 684 and corresponding vent(s) in the circuit board. As described herein, the electronics assembly 600 can be embedded within layers of the dressing, such as in cutouts or recesses into which the electronics assembly can be placed.

The pump inlet protection mechanism 610 can provide a large surface area available for vacuum to be drawn by the inlet of the pump. A pump inlet (shown as rounded protrusion in FIG. 7A) can fit within a recess in the pump inlet protection mechanism 610. The pump inlet can be attached by friction fit and/or form a complementary fit to the recess of the pump inlet protection mechanism.

The pump inlet protection mechanism 610 can allow air or gas to pass through, but can block liquid from reaching the negative pressure source. The pump inlet protection mechanism 610 can include a porous material. The pump inlet protection mechanism 610 can comprise one or more porous polymer molded components. The pump inlet protection mechanism 610 can include hydrophobic or substantially hydrophobic material. Material included in the pump inlet protection mechanism 610 can have a pore size in the range of approximately 5 microns to approximately 40 microns. The pore size can be approximately 10 microns. In some cases, the pump inlet protection mechanism 610 can include a polymer that can be one of hydrophobic polyethylene or hydrophobic polypropylene. In some cases, the pump inlet protection mechanism can include a Porvair Vyon material with a pore size of 10 microns. Any of the pump inlet protection mechanism described herein can include one or more features of the pump inlet protection mechanism 610.

### Canisterless Pump Assembly

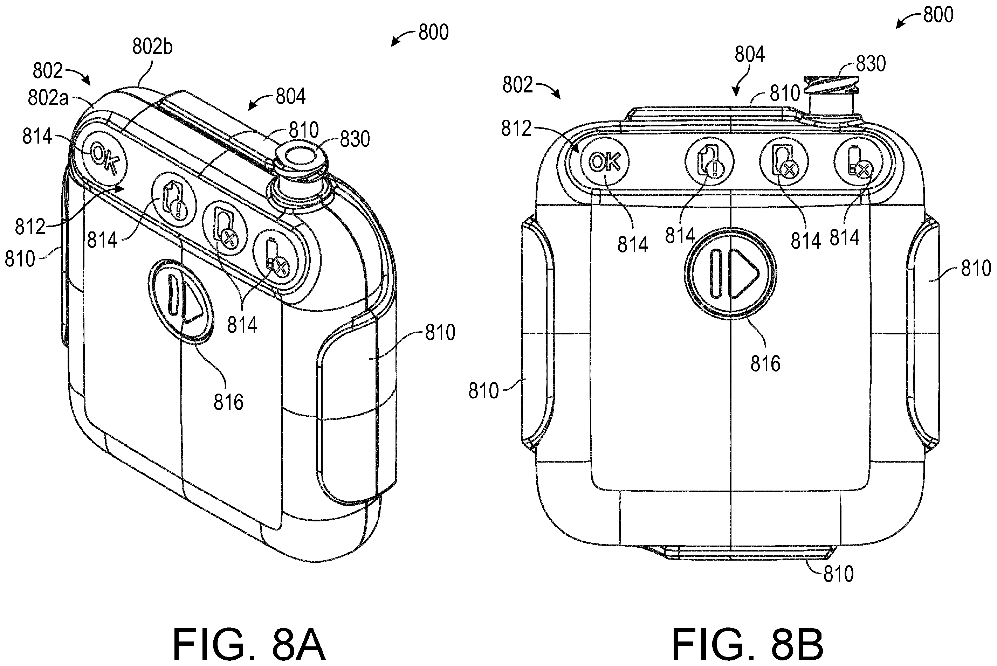

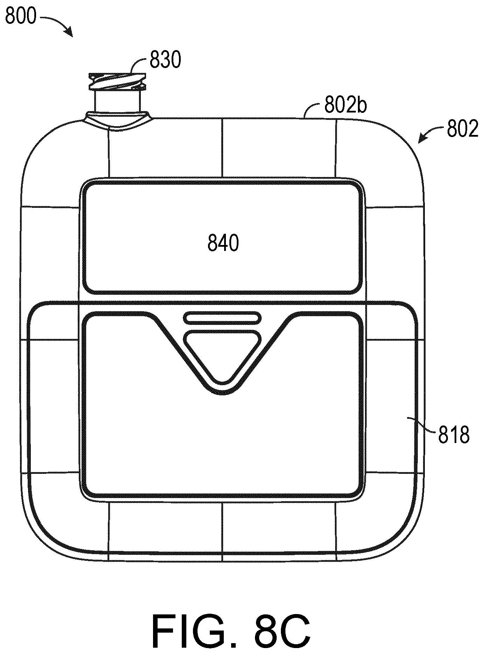

Figures 8A-8C illustrate perspective, front, and rear views of a reduced pressure wound therapy device 800. The reduced pressure wound therapy device 800 can include a housing 802 and an optional mounting component 810. The mounting component (or attachment) 810 can be removably attached to the housing 802, such that the reduced pressure wound therapy device 800 can be used with or without the mounting component 810. For example, Figure 8C illustrates the reduced pressure wound therapy device 800 without the mounting component 810. The mounting component 810 can be designed to allow the reduced pressure wound therapy device 800 to be mounted on another object such as, but not limited to, a user's person. The mounting component 810 can include a clip designed to retain the mounting component 810 on a user's outerwear, such as on a user's pocket, a pouch, a belt, a flap, or otherwise.

The housing 802 (sometimes referred to as "outer housing") can contain or support components of device reduced pressure wound therapy device 800. The housing 802 can be formed from one or more portions, such as a front portion 802a and a rear portion 802b, which can be removably attached to form the housing 802.

The housing 802 can include a user interface 812 which can be designed to provide a user with information (for example, information regarding an operational status of the reduced pressure wound therapy device 800). The user interface 812 can include one or more indicators, such as icons 814, which can alert the user to one or more operating or failure conditions of the reduced pressure wound therapy system. For example, the indicators can include icons for alerting the user to normal or proper operating conditions, pump failure, power failure, the condition or voltage level of the batteries, the condition or capacity of a wound dressing, detection of a leak within the wound dressing or fluid flow pathway between the wound dressing and the pump assembly, suction blockage, or any other similar or suitable conditions or combinations thereof. An example set of icons 814 is illustrated in Figures 8A-8B which, from left to right, can include an "OK" indicator which can indicate normal operation of the system, a "leak" indicator which can indicate the existence of a leak in the system, a "dressing full" indicator which can indicate that a wound dressing is at or near capacity, and a "battery critical" indicator which can indicate that the power source (such as, one or more batteries) is at or near a critical level. The icons 814 can have a green or orange color, or can be illuminated with a green or orange light (for example, colored LEDs).

The reduced pressure wound therapy device 800 can include one or more user input features, such as button 816, designed to receive an input from the user for controlling the operation of the device 800. A single button can be present which can be used to activate and deactivate the reduced pressure wound therapy device or control other operating parameters of the device 800. For example, the button 816 can be used to activate the recued pressure wound therapy device 800, pause the device 800, clear indicators (such as, one or more icons 814, or be used for any other suitable purpose for controlling an operation of the device 800 (for example, by sequentially pushing on the button 816). The button 816 can be a push style button that can be positioned on an outside, front surface of the housing 802. In some cases, multiple input features (for example, multiple buttons) can be provided.

The reduced pressure wound therapy device 800 can include a connector 830 for connecting a tube or conduit to the device 800. The connector 830 can be used to connect reduced pressure wound therapy device to a wound dressing.

The reduced pressure wound therapy device 800 can be a canisterless device. The wound dressing can retain fluid (such as, exudate) aspirated from the wound. Such a dressing can include a filter, such as a hydrophobic filter, that prevents passage of liquids downstream of the wound dressing (toward the reduced pressure wound therapy device 800).

The reduced pressure wound therapy device 800 can include a removable cover 818, as illustrated in Figure 8C. The cover 818 can cover a cavity (not shown) in which one or more power sources, such as batteries, for powering the device 800 are positioned.

Any of the negative pressure wound therapy devices described herein can include one or more features disclosed in U.S. Patent Publication No. 2019/0231939.

### Multiple Negative Pressure Sources

Some pumps (for example, those with an electromechanical motor) may generate an audible noise and/or vibration as they operate. The generated audible noise and/or vibration can be a source of discomfort and/or inconvenience to a patient, so much so that, in some cases, this can cause a patient (or caregiver) to deactivate the pump. This can result in one or more undesirable interruptions in the application of negative pressure wound therapy, which may extend or even compromise the healing process. In many circumstances, it is desirable for the pump to remain active, for example so that the pump may be utilized for applying negative pressure wound therapy to the patient. For at least these reasons, it may be advantageous to utilize a pump that does not produce (or produces less) audible noise and/or vibration.

One such example of a pump that does not produce (or produces less) audible noise and/or vibration is a piezoelectric pump. For example, a piezoelectric pump can include a piezoelectric transducer that is caused to vibrate or oscillate in response to receiving an electrical signal (sometimes referred to a driving signal). The driving signal can be an alternating current (AC) signal. Because a piezoelectric transducer can oscillate at frequencies that are outside the range of human hearing (such as at frequencies above 20 kHz), the audio noise produced by the piezoelectric pump can be less noticeable (for example, from the perspective of the patient). In addition, because a piezoelectric transducer can operate efficiently without causing excessive vibration (for example, when the transducer is driven at its resonant frequency or mechanical resonant frequency), vibration of the pump can be reduced.

While piezoelectric pumps many have many advantages, including a capability of being quieter as described above, piezoelectric pumps can have moderate pressure delivery capabilities, as compared to other pumps. For example, in some cases, piezoelectric pumps can deliver a maximum pressure of about -80 mmHg or about -100 mmHg. Although the maximum deliverable pressure of a piezoelectric pump may be moderate, it nonetheless can be advantageous for a NPWT system to utilize a piezoelectric pump at least for the reasons described herein.

In some cases, a NPWT system can include multiple pumps (for example, piezoelectric pumps), such as 2, 3, 4, or more pumps. For example, the multiple pumps can be pneumatically connected in series, which can have an effect of additively combining the pressure generated by each pump. Thus, a NPWT system having multiple pumps can generate a threshold pressure (which may correspond to the negative pressure setpoint) that exceeds the maximum pressure generating capability of a NPWT system having only a single pump. Furthermore, in some cases, the multiple pumps of the NPWT system can be relatively quiet so as to not produce excessive noise as described herein.

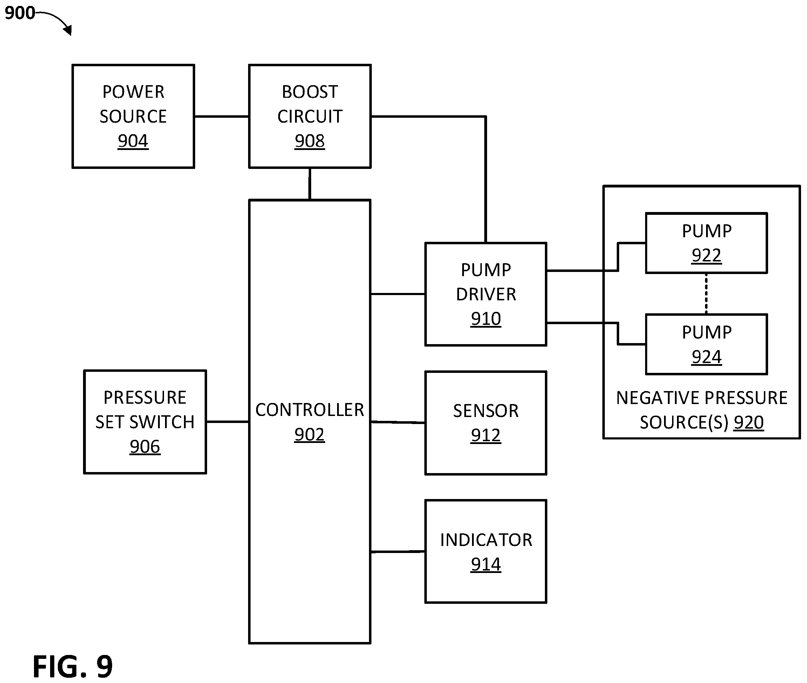

FIG. 9 illustrates an example NPWT system 900 that includes multiple pumps or negative pressure sources. The NPWT system 900 can be used to treat a wound and can be similar to any of the other systems described herein. The NPWT system 900 can include one or more of: multiple negative pressure sources 920 (two such sources 922 and 924 are illustrated), a controller 902, a power source 904, a pressure set switch 906, a boost circuit 908, a pump driver 910, a sensor 912, or an indicator 914. It will be understood that the NPWT system 900 can include fewer, more, or different components as desired.

The power source 904 can provide power to one or more components of the NPWT system 900. The power source 904 can include one or more power supplies, such as batteries (for example, multiple 3V batteries), to provide power for one or more components of the NPWT system 900. The power source 904 can, for instance, provide electrical energy (e.g., current or voltage) to the boost circuit 908. In some cases, a voltage output by the power source 904 to the boost circuit 908 can be around 6 V. In some cases, the power source 904 can include additional circuitry, such as the boost circuit 908, or can be in electrical communication with one or more other components of the NPWT system 900, such as the controller 902, the pump driver 910, etc.

The boost circuit 908 can be in electrical communication with one or more of the power source 904, the controller 902, or the pump driver 910. In some cases, the boost circuit 908 can control the electrical current or voltage received from the power source 904 or provided to the pump driver 910. For example, in some cases, the boost circuit 908 can function as a boost converter to boost or increase voltage or current from the power source 904. For instance, in some cases, the boost circuit 908 can boost or increase a voltage received from the power source 904 to a threshold voltage level, such as to between 20 V and 30 V (for example, or about 28 V). As another example, in some cases, the boost circuit 908 can serve to limit the current or clamp the current at a threshold current level, such as at 90 mA, 250 mA, 466 mA, 500 mA, or 1 A. In some cases, the boost circuit 908 can limit potential fault current, for example through the pump driver 910, the negative pressure sources 920, etc.

The sensor 912 can include one or more of various sensors, such as, but not limited to, a sensor for pressure, temperature (e.g., a thermistor sensor), tissue color (e.g., an optical sensor), pH, conductivity or impedance, or the like. For example, the sensor 912 can include any of the sensors described herein, such as any of pressure sensors 691 and 692 of FIG. 6B.

The indicator 914 can include one or more of various indicators, such as, but not limited to one or more of a visual, audio, or tactile indicators. In some cases, indicator 914 can be any of the indicators described herein, such as one or more of the indicators 202 or 204 of FIG. 2. In some cases, the indicator 904 can be configured to indicate alarms or indicate status of the NPWT system 900. In some cases, the indicator 914 can be configured to alert a user, such as patient or medical care provider, to a variety of operating or failure conditions of the NPWT system 900, including alerting the user to normal or proper operating conditions, pump failure, power supplied to the pump or power failure, detection of a leak, suction blockage, no flow condition, canister full condition, or any other similar or suitable conditions or combinations thereof.

The pressure set switch 906 can be utilized to provide or adjust the pressure setpoint.

The negative pressure sources 920 can be configured to provide negative pressure, for example, to aspirate fluid from a wound. One or more of the negative pressure sources 920 can be disposed on or within a wound dressing, as described herein. The negative pressure sources 920 can be pneumatically connected in series. In some cases, being pneumatically connected in series can have an effect of additively combining the pressure generated by each pump 922 and 924. The negative pressure sources 920 can be electrically connected in parallel. In some cases, being electrically connected in parallel allows each of the negative pressure sources 920 to receive the same (or similar) drive signal from the pump driver 910. However, it will be understood that, in some cases, one or more of the negative pressure sources 920 can be pneumatically connected in parallel and/or electrically connected in series.

As illustrated, the negative pressure sources 920 can include a plurality of negative pressure sources 920. In the illustrated example of FIG. 9, the negative pressure sources 920 includes a first pump 922 and a second pump 924. However, it will be understood that, in some cases, the NPWT system 900 can include additional negative pressure sources, such as a total of three, four, or more negative pressure sources. Any of the negative pressure sources 920 can be the same or similar as, or include any of the same or similar features as, any of the negative pressure sources described herein, such as pump 272 of FIG. 2A or pump 672 of any of FIGS. 6A-6B and 7A-7B.

Some or all of the negative pressure sources 920 can be in electrical communication with the pump driver 910. For example, as described herein, each of the negative pressure sources 920 can receive a drive signal from the pump driver 910. In some cases, the each of the negative pressure sources 920 receives the same (or similar) drive signal from the pump driver 910. In some cases, two or more of negative pressure sources 920 receive a different drive signal from the same pump driver 910. In some cases, two or more of negative pressure sources 920 receive a different drive signal from the different pump drivers 910.

In some cases, the negative pressure sources 922 and 924 can include one or more piezoelectric transducers. Each of the piezoelectric transducers of the negative pressure sources 920 have an associated mechanical resonant frequency (as well as, in some cases, one or more subharmonic or harmonic frequencies). The resonant frequency can correspond to a frequency at which a piezoelectric transducer most efficiently converts the electrical energy provided by the driving signal to mechanical energy of oscillation of the transducer. Such energy conversion can be additionally or alternatively referred to as power transfer. For example, each of the negative pressure sources 920 can have a pump-specific mechanical resonant frequency, which can correspond to an optimum or near optimum efficiency level of the particular negative pressure source. The mechanical resonant frequency of a particular negative pressure source can be attributable to, for example, operating temperatures, manufacturing differences of the negative pressure source, and/or the mechanical/electrical design (e.g., dimensions of a piezoelectric component) of the negative pressure source,. Furthermore, in some implementations, the mechanical resonant frequency of a negative pressure source can vary in response to, for example, variations in temperature, humidity, and the like. Thus, the mechanical resonant frequencies can vary amongst the different negative pressure sources 920. In some cases, the mechanical resonant frequency of any of the negative pressure sources 920 can be between 5 KHz and 100 kHz, such as around 20 KHz, 22 kHz, or 24 kHz or greater or less than 5 KHz and 100 kHz.

In some cases, the mechanical resonant frequency may not be known in advance of operating the NPWT system 900 or known with a high precision or accuracy. In some cases, as described herein, the NPWT system 900 can tune the frequency of the drive signal applied by the pump driver 910 to the negative pressure sources to substantially match the mechanical resonant frequency, or a function of the mechanical resonant frequency, of one or more of the negative pressure sources. Moreover, the frequency (and/or magnitude) of the drive signal provided to the negative pressure sources can be monitored and/or updated (for example, based on the mechanical resonant frequency of one or more of the negative pressure sources), as the mechanical resonant frequencies may change due to the changing operating conditions. For example, the operating conditions can include changes in the temperature, duration of operation, humidity, or the like. The monitoring and/or updating of the frequency (and/or magnitude) of the drive signal can be performed periodically.

The pump driver 910 can be in electrical communication with the boost circuit 908, the controller 902, and the negative pressure sources 920. For example, the pump driver 910 can receive energy (e.g., voltage or current) from the boost circuit 908 and control signals from the controller 902 in order to generate output drive signals to the negative pressure sources 920. Although FIG. 9 is illustrated as including one pump driver 910, in some cases, the NPWT system 900 can include multiple pump drivers 910. For example, in some cases, the NPWT system 900 can include a designated pump driver 910 for each of the negative pressure sources 920.

In some cases, the pump driver 910 can control the supply of negative pressure produced by the negative pressure sources 920. For example, the pump driver 910 can provide one or more drive signals in the form of one or more electrical currents or voltages to each of the negative pressure sources 920 (for example, to the piezoelectric transducer of the first pump 922 and to the piezoelectric transducer of the second pump 924). The pump driver 910 can, for instance, generate and provide AC electrical signal to the first pump 922 and the second pump 924. In some cases, applying the drive signal to the negative pressure sources 920 results in positive charge flowing away from the pump driver 910 (that is, sourcing of electrical current by the pump driver 910) or toward the pump driver 910 (that is, sinking of electrical current by the pump driver 910).

In some cases, the pump driver 910 can apply the same or a similar drive signal to each of the negative pressure sources 920. For example, the pump driver 910 can generate a first drive signal with a first magnitude and a first frequency, and the pump driver 910 can supply the first drive signal to each of the first pump 922 and the second pump 924. In some cases, the pump driver 910 can supply a different drive signal to each of the negative pressure sources 920. For example, the pump driver 910 can generate a first drive signal with a first magnitude and a first frequency, and can generate a second drive signal with a second magnitude and a second frequency, and the pump driver 910 can supply the first drive signal to the first pump 922 and the second drive signal to the second pump 924. The first and second magnitudes can be the same or different. The first and second frequencies can be the same or different.

In some cases, the pump driver 910 can apply the same or a similar drive signal to each of the negative pressure sources 920. For example, the same or similar drive signal can include a drive signal with the same frequency or the same magnitude. In some cases, the frequency (and/or magnitude) of the drive signal applied to the negative pressure sources 910 can be based on the mechanical resonant frequency of one or more of the negative pressure sources 910. As described herein, some or all of the negative pressure sources 910 can have different mechanical resonant frequencies. In some cases, the frequency (and/or magnitude) of the drive signal applied to the negative pressure sources 910 can be a function of one or more of the mechanical resonant frequencies. For example, the frequency (and/or magnitude) of the drive signal can be an average or a median of the mechanical resonant frequency of two or more of the negative pressure sources 910. As another example, in some cases, the frequency (and/or magnitude) of the drive signal applied to the negative pressure sources 910 can correspond to the mechanical resonant frequency of one of the negative pressure sources 910, such as the mechanical resonant frequency of the first pump 922.

In some cases, the pump driver 910 applies a drive signal to the negative pressure sources 910 that will maximize a combined amount of power transferred to the negative pressure sources 910 (at least when considering the constraint of applying the same drive signal to each of the negative pressure sources 910). This can improve efficiency of the NPWT system 900 (which, for example, can be measured by a more efficient power consumption). For example, although a power transferred to a particular negative pressure source may not be maximized (for example, because one or more of the magnitude or frequency of the drive signal does not match the mechanical resonant frequency of that particular negative pressure source), the aggregate power provided to the negative pressure sources 910 can be optimum or near optimum (at least when considering the constraint of applying the same drive signal to each of the negative pressure sources 910). In some cases, to determine the drive signal parameters (e.g., frequency, magnitude, or phase of the drive signal), the controller 902 alone or in combination with one or more of the boost circuit 908 or the pump driver 910, can tune the drive signal over time to determine the drive signal parameters that will maximize the combined amount of power transferred to the negative pressure sources 910.

In some cases, the pump driver 910 can apply a pump-specific drive signal to each of the negative pressure sources 910. For example, the pump driver 910 can generate different drive signals for each of the negative pressure sources 910. In some cases, the drive signal applied to a particular negative pressure source correspond to the mechanical resonant frequency of that particular negative pressure source, which can maximize a combined amount of power transferred to the negative pressure sources 910. In this way, the NPWT system 900 improves efficiency, as each negative pressure source is supplied a drive signal having parameters that will maximize the amount of power transferred to that particular negative pressure source.

In some cases, to determine the drive signal parameters (e.g., the particular frequency, magnitude, or phase), the pump driver 910, the controller 902, or a combination thereof, can tune the drive signals over time to determine the drive signal parameters that will maximize the amount of power transferred to each negative pressure source. In some cases, to provide the capability of supplying different drive signals to each of the negative pressure sources 910, the NPWT system 900 can include a different pump driver 910 for each negative pressure source. For example, the pump driver 910 can have any of the features, or perform any of the steps or methods, described disclosed in U.S. Patent Publication No. 2019/0143007, entitled "Optimizing Power Transfer To Negative Pressure Sources In Negative Pressure Therapy Systems".

In some cases, the pump driver 910 can include an H-bridge circuit composed of multiple switches. In some cases, the pump driver 910 can include multiple H-bridge circuits, such as a designated H-bridge circuit for each negative pressure source. The H-bridge circuit(s) can be constructed to operate as an H-bridge inverter. As a non-limiting example and with reference to the example of FIG. 9, the controller 902 can generate first and second control signals to cause the H-bridge circuit(s) of the pump driver 910 to output electrical currents and voltages having a square waveform (such as about ± 30 V) with a frequency (such as 18 kHz to 24 kHz or about 21 kHz) and a duty cycle or ratio (such as about 50%), which can be provided to the first pump 922 and the second pump 924, respectively.

The controller 902 can be in electrical communication with any of the pressure set switch 906, the indicator 914, the sensor 912, the pump driver 910, the boost circuit 908, the power source 904, the negative pressure sources 920, etc. In some cases, the controller 902 can control the supply negative pressure produced by the negative pressure sources 920. For example, in some cases, the controller 902 can control operation of the pump driver 910, and, in turn, the negative pressure sources 920, by outputting one or more control signals to the negative pressure sources 920 via the pump driver 910. In some cases, the controller 902 can vary characteristics (for example, a pulse width modulation (PWM), frequency, magnitude, etc.) of the one or more control signals provided to the pump driver 910, thereby varying the drive signal (for example, an electrical current or voltage) provided by the pump driver 910 to the negative pressure sources 920.

In some cases, the pump driver 910 can provide feedback to the controller 902, and the controller 902 can, in turn, control the operations of the pump driver 910 based on the feedback. For example, in some cases, the pump driver 910 and the controller 902 can perform an iterative process to tune the transfer of power (for example, by adjusting parameters of a drive signal such as frequency, magnitude, or phase) to the negative pressure sources 920, as described herein. Furthermore, in some cases, the controller 902 can operate the pump driver 910 to increase or decrease the frequency of the drive signal applied by the pump driver 910 to the negative pressure sources 920. In some cases, the controller 902 can operate the pump driver 910 to increase or decrease the frequency of the drive signal to tune the drive signal such that its frequency does not satisfy a threshold frequency, such as one that is not a subharmonic or harmonic frequency associated with a negative pressure source.

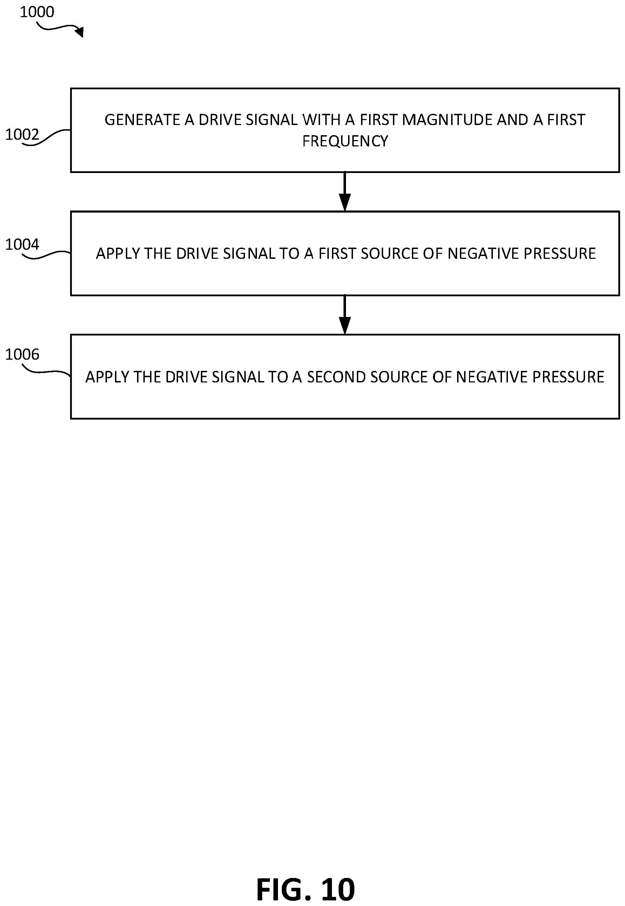

FIG. 10 illustrates a process 1000 for operating a NPWT system. In some cases, the blocks outlined for the process 1000 can be implemented by the NPWT system 900 of FIG. 9, such as by the controller 902 alone or in combination with, for example, the pump driver 910. For ease of reference, the process 1000 can be assumed as being generally performed by the controller 902 in combination with the pump driver 910. However, this assumption should not be construed as limiting.

At block 1002, the process 1000 can generate a drive signal. In some cases, the drive signal can be in the form of an AC signal. As described herein, the drive signal can be generated to have a particular frequency, such as a first frequency. As described herein, the frequency (or other parameters) of the drive signal can be based at least in part on a resonant frequency associated with one or more of the negative pressure sources 920. In some cases, to generate the drive signal, or to determine the frequency of the drive signal, the pump driver 910, the controller 902, or a combination thereof, performs a process of tuning the drive signal. For example, the process 100 can iteratively tune one or more of frequency, magnitude, or phase of the drive signal until the aggregate power provided to the negative pressure sources 910 is optimum or near optimum (at least when considering the constraint of applying the same drive signal to each of the negative pressure sources 910). That is, the process 100 can determine one or more of a frequency, magnitude, or phase that will maximize the combined amount of power transferred to the negative pressure sources 910, at least when considering the constraint of applying the same drive signal to each of the negative pressure sources 910.

At block 1004, the process 1000 can apply the drive signal to a first pump 922. As described herein, the first pump 922 can be disposed on or within a wound dressing, such as any wound dressing described herein. The first pump 922 can include a first piezoelectric transducer. In some cases, the wound dressing can be placed over a wound of a patient and the wound dressing can absorb fluid, such as fluid from the wound. In some cases, the first pump 922 can be located remote or separate from the wound dressing.

At block 1006, the process 1000 can apply the drive signal to a second pump 924. As described herein, the second pump 924 can be disposed on or within the wound dressing, such as the same wound dressing in which the first pump 922 is disposed. The second pump 924 can include a second piezoelectric transducer. In some cases, second pump 924 can be located remote or separate from the wound dressing.

As described herein, in some cases, the first pump 922 and second pump 924 are pneumatically connected in series. In some such cases, the maximum level of negative pressure capable of being produced by the combination of the first pump 922 and the second pump 924 can be greater than individual maximum levels of negative pressure of the first pump 922 or the second pump 924. As an example, if the individual maximum level of negative pressure of the first pump 922 is -100 mmHg and the individual maximum level of negative pressure of the second pump 924 is -100 mmHg, then the maximum level of negative pressure capable of being produced by the combination of the first pump 922 and the second pump 924 can, in some cases, be about -180 mmHg, about -190 mmHg, or about -200 mmHg.

As described herein, the first pump 922 can be associated with a first resonant frequency, and the second pump 924 can be associated with a second resonant frequency that is different from the first resonant frequency. In some cases, the first drive signal has a drive frequency (and/or magnitude) that is different from the first resonant frequency and the second resonant frequency (and/or magnitude). In some cases, the first drive signal has a frequency (and/or magnitude) that is the same or substantially similar to first resonant frequency or the second resonant frequency (and/or magnitude). In some cases, the first drive signal has a frequency (and/or magnitude) that is a function of the first resonant frequency or the second resonant frequency (and/or magnitude). For example, the frequency (and/or magnitude) of the drive signal can be an average of the first resonant frequency and the second resonant frequency (and/or magnitude).

It will be understood that the various blocks described herein with reference to Fig. 10 can be implemented in a variety of orders. Furthermore, the system 1000 can implement some blocks concurrently or change the order as desired. For example, in some cases, the process 1000 can concurrently apply the drive signal to the first pump 922 and the second pump 924 by combining the blocks 1004 and 1006.

Furthermore, it will be understood that fewer, more, or different blocks can be used as part of the process 1000. For example, the process 1000 can generate a different drive signal for each negative pressure source. For example, as described herein, the process 1000 can generate a first drive signal that has a first frequency (and/or magnitude) which matches or is substantially similar to the first resonant frequency (and/or magnitude) of the first pump 922, and can generate a second drive signal that has a second frequency (and/or magnitude) which matches or is substantially similar to the second resonant frequency (and/or magnitude) of the second pump 924. Further, at block 1004, the process 1000 can apply the first drive signal to the first pump 922, and at block 1006, the process 1000 can apply the second drive signal to the second pump 924.

### Other Variations

While piezoelectric pumps have been described as one example, other pump(s) can be additionally or alternatively utilized, including voice coil pump(s). While certain embodiments described herein relate to integrated negative pressure wound therapy systems in which the negative pressure source is supported by the dressing, systems and methods described herein are applicable to any negative pressure wound therapy system or medical system. For example, systems and methods for extending operational time described herein can be used in negative pressure wound therapy systems or medical systems. Such systems can be configured with the negative pressure source and/or electronics being external to the wound dressing. Additionally, the systems and methods disclosed herein can be utilized by ultrasound delivery devices, negative pressure devices powered by an external power supply (including PICO device manufactured by Smith & Nephew), negative pressure devices with a separate pump, and medical devices generally.

Any of the embodiments disclosed herein can be used with one or more features disclosed in U.S. Patent No. 7,779,625, titled "DEVICE AND METHOD FOR WOUND THERAPY," issued August 24, 2010; U.S. Patent No. 7,964,766, titled "WOUND CLEANSING APPARATUS IN SITU," issued on June 21, 2011; U.S. Patent No. 8,235,955, titled "WOUND TREATMENT APPARATUS AND METHOD," issued on August 7, 2012; U.S. Patent No. 7,753,894, titled "WOUND CLEANSING APPARATUS WITH STRESS," issued July 13, 2010; U.S. Patent No. 8,764,732, titled "WOUND DRESSING," issued July 1, 2014; U.S. Patent No. 8,808,274, titled "WOUND DRESSING," issued August 19, 2014; U.S. Patent No. 9,061,095, titled "WOUND DRESSING AND METHOD OF USE," issued June 23, 2015; US Patent No. 10,076,449, issued September 18, 2018, titled "WOUND DRESSING AND METHOD OF TREATMENT"; U.S. Patent Application No. 14/418908, filed January 30, 2015, published as U.S. Publication No. 2015/0190286, published July 9, 2015, titled "WOUND DRESSING AND METHOD OF TREATMENT"; U.S. Patent No. 10,231,878, titled "TISSUE HEALING," issued March 19, 2019; PCT International Application PCT/GB2012/000587, titled "WOUND DRESSING AND METHOD OF TREATMENT" and filed on July 12, 2012; International Application No. PCT/IB2013/001469, filed May 22, 2013, titled "APPARATUSES AND METHODS FOR NEGATIVE PRESSURE WOUND THERAPY"; PCT International Application No. PCT/IB2013/002102, filed July 31, 2013, titled "WOUND DRESSING AND METHOD OF TREATMENT"; PCT International Application No. PCT/IB2013/002060, filed July 31, 2013, titled "WOUND DRESSING AND METHOD OF TREATMENT"; PCT International Application No. PCT/IB2013/00084, filed March 12, 2013, titled "REDUCED PRESSURE APPARATUS AND METHODS"; International Application No. PCT/EP2016/059329, filed April 26, 2016, titled "REDUCED PRESSURE APPARATUSES"; PCT International Application No. PCT/EP2017/059883, filed April 26, 2017, titled "WOUND DRESSINGS AND METHODS OF USE WITH INTEGRATED NEGATIVE PRESSURE SOURCE HAVING A FLUID INGRESS INHIBITION COMPONENT"; PCT International Application No. PCT/EP2017/055225, filed March 6, 2017, titled "WOUND TREATMENT APPARATUSES AND METHODS WITH NEGATIVE PRESSURE SOURCE INTEGRATED INTO WOUND DRESSING"; PCT International Application No. PCT/EP2018/074694, filed September 13, 2018, titled "NEGATIVE PRESSURE WOUND TREATMENT APPARATUSES AND METHODS WITH INTEGRATED ELECTRONICS"; PCT International Application No. PCT/EP2018/074701, filed September 13, 2018, titled "NEGATIVE PRESSURE WOUND TREATMENT APPARATUSES AND METHODS WITH INTEGRATED ELECTRONICS"; PCT International Application No. PCT/EP2018/079345, filed October 25, 2018, titled "NEGATIVE PRESSURE WOUND TREATMENT APPARATUSES AND METHODS WITH INTEGRATED ELECTRONICS"; PCT International Application No. PCT/EP2018/ 079745, filed October 30, 2018, titled "SAFE OPERATION OF INTEGRATED NEGATIVE PRESSURE WOUND TREATMENT APPARATUSES".

Although the present invention as claimed concerns a negative pressure wound therapy system and is related to wound dressings, systems and methods disclosed herein are not limited to wound dressings or medical applications. Systems and methods disclosed herein are generally applicable to electronic devices in general, such as electronic devices that can be worn by or applied to a user.

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes may be described in terms of determining whether a value meets or does not meet a particular threshold, the blocks can be similarly understood, for example, in terms of a value (i) being below or above a threshold or (ii) satisfying or not satisfying a threshold.

Features, materials, characteristics, or groups described in conjunction with a particular aspect, embodiment, or example are to be understood to be applicable to any other aspect, embodiment or example described herein unless incompatible therewith.

While certain embodiments have been described, these embodiments have been presented by way of example only, and are not intended to limit the scope of protection, which is defined by the appended claims. The disclosed methods and systems described herein may be embodied in a variety of other forms. Furthermore, various omissions, substitutions and changes in the form of the methods and systems described herein may be made. Those skilled in the art will appreciate that in some cases, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure.

The various components illustrated in the figures may be implemented as software or firmware on a processor, controller, ASIC, FPGA, or dedicated hardware. Hardware components, such as controllers, processors, ASICs, FPGAs, and the like, can include logic circuitry. Furthermore, the features and attributes of the specific embodiments disclosed above may be combined in different ways to form additional embodiments, all of which fall within the scope of the present disclosure.

Although the present disclosure includes certain embodiments, examples and applications, it will be understood by those skilled in the art that the scope of protection is defined by the appended claims.

Conditional language, such as "can," "could," "might," or "may," unless specifically stated otherwise, or otherwise understood within the context as used, is generally intended to convey that certain embodiments include, while other cases do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount.

## Claims

1. A negative pressure wound therapy system (900) comprising:
a first source of negative pressure (922) configured to supply negative pressure to a wound covered by a wound dressing; and
a second source of negative pressure (924) configured to supply negative pressure to the wound covered by the wound dressing, the second source of negative pressure (924) pneumatically connected in series with the first source of negative pressure (922), wherein the first source of negative pressure (922) comprises a first piezoelectric transducer and the second source of negative pressure (924) comprises a second piezoelectric transducer, wherein the first piezoelectric transducer has a first resonant frequency and the second piezoelectric transducer has a second resonant frequency;
characacterized in that
the system comprises electronic circuitry configured to:
generate a first driving signal with a first driving signal magnitude and a first driving signal frequency, wherein the first driving signal frequency corresponds to an average of the first resonant frequency and the second resonant frequency;
generate a second driving signal with a second driving signal magnitude and a second driving signal frequency, wherein the second driving signal frequency corresponds to an average of the first resonant frequency and the second resonant frequency; and
apply the first driving signal to the first piezoelectric transducer of the first source of negative pressure (922) and apply the second driving signal to the second piezoelectric transducer of the second source of negative pressure (924) and cause the first and second sources of negative pressure (922, 924) to provide negative pressure collectively at a maximum negative pressure level that is greater than individual maximum negative pressure levels of the first and second sources of negative pressure (922, 924).

2. The system (900) of claim 1, further comprising a housing enclosing the first and second sources of negative pressure (922, 924) and the electronic circuitry.

3. The system (900) of any one of the preceding claims, wherein the maximum negative pressure level is equal to a combined individual maximum negative pressure level of the first and second sources of negative pressure (922, 924).

4. The system (900) of any one of the preceding claims, wherein the electronic circuitry is further configured to determine the first and second resonant frequencies at initialization of the system, monitor current first and second resonant frequencies during operation of the system, and in response to a determination that at least one of the first or second current resonant frequencies is different from a corresponding resonant frequency determined at the initialization of the system, set a corresponding driving signal frequency to the current resonant frequency.

5. The system (900) of any one of the preceding claims, wherein the second resonant frequency different from the first resonant frequency.

6. The system (900) of any one of the preceding claims, wherein the electronic circuity comprises a first driving circuitry configured to actuate the first source of negative pressure and a second driving circuitry configured to actuate the second source of negative pressure.

7. The system (900) of any one of the preceding claims, comprising:
a wound dressing configured to be placed over a wound of a patient, the wound dressing configured to absorb fluid;
wherein the first source of negative pressure (922) is disposed on or within the wound dressing,
wherein the second source of negative pressure (924) is disposed on or within the wound dressing,
wherein the electronic circuitry is disposed on or within the wound dressing, wherein the electronic circuitry is configured to cause the first and second sources of negative pressure (922, 924) to provide negative pressure to aspirate fluid from the wound.

8. The system (900) of claim 7, wherein applying the first driving signal to the first piezoelectric transducer of the first source of negative pressure (922) and applying the second driving signal to the second piezoelectric transducer of the second source of negative pressure (924) pneumatically connected in series causes provision of negative pressure to the wound at a maximum negative pressure level that is greater than individual maximum negative pressure levels of the first and second sources of negative pressure (922, 924).

9. The system (900) of claim 8, wherein the maximum negative pressure level is equal to a combined individual maximum negative pressure level of the first and second sources of negative pressure (922, 924).

## Patentansprüche

1. Unterdruck-Wundbehandlungssystem (900), umfassend:
eine erste Unterdruckquelle (922), die dazu ausgelegt ist, einer durch einen Wundverband bedeckten Wunde Unterdruck zuzuführen; und
eine zweite Unterdruckquelle (924), die dazu ausgelegt ist, der durch den Wundverband bedeckten Wunde Unterdruck zuzuführen, wobei die zweite Unterdruckquelle (924) pneumatisch in Reihe mit der ersten Unterdruckquelle (922) geschaltet ist, wobei die erste Unterdruckquelle (922) einen ersten piezoelektrischen Wandler umfasst und die zweite Unterdruckquelle (924) einen zweiten piezoelektrischen Wandler umfasst, wobei der erste piezoelektrische Wandler eine erste Resonanzfrequenz aufweist und der zweite piezoelektrische Wandler eine zweite Resonanzfrequenz aufweist;
**dadurch gekennzeichnet, dass**
das System eine elektronische Schaltungsanordnung umfasst, die ausgelegt ist zum:
Erzeugen eines ersten Ansteuersignals mit einer ersten Ansteuersignalgröße und einer ersten Ansteuersignalfrequenz, wobei die erste Ansteuersignalfrequenz einem Mittelwert von erster Resonanzfrequenz und zweiter Resonanzfrequenz entspricht;
Erzeugen eines zweiten Ansteuersignals mit einer zweiten Ansteuersignalgröße und einer zweiten Ansteuersignalfrequenz, wobei die zweite Ansteuersignalfrequenz einem Mittelwert von erster Resonanzfrequenz und zweiter Resonanzfrequenz entspricht; und
Anlegen des ersten Ansteuersignals an den ersten piezoelektrischen Wandler der ersten Unterdruckquelle (922) und Anlegen des zweiten Ansteuersignals an den zweiten piezoelektrischen Wandler der zweiten Unterdruckquelle (924) und Bewirken, dass die erste und die zweite Unterdruckquelle (922, 924) gemeinsam Unterdruck mit einem maximalen Unterdruckpegel bereitstellen, der größer als die einzelnen maximalen Unterdruckpegel der ersten und der zweiten Unterdruckquelle (922, 924) ist.

2. System (900) nach Anspruch 1, ferner ein Gehäuse umfassend, das die erste und die zweite Unterdruckquelle (922, 924) und die elektronische Schaltungsanordnung umschließt.

3. System (900) nach einem der vorhergehenden Ansprüche, wobei der maximale Unterdruckpegel gleich einem kombinierten einzelnen maximalen Unterdruckpegel von erster und zweiter Unterdruckquelle (922, 924) ist.

4. System (900) nach einem der vorhergehenden Ansprüche, wobei die elektronische Schaltungsanordnung ferner dazu ausgelegt ist, die erste und die zweite Resonanzfrequenz bei Initialisierung des Systems zu bestimmen, die erste und die zweite Resonanzfrequenz des Stroms während des Betriebs des Systems zu überwachen und als Reaktion auf eine Bestimmung, dass mindestens eine von erster oder zweiter Resonanzfrequenz des Stroms von einer entsprechenden Resonanzfrequenz verschieden ist, die bei der Initialisierung des Systems bestimmt wird, eine entsprechende Ansteuersignalfrequenz auf die Resonanzfrequenz des Stroms einzustellen.

5. System (900) nach einem der vorhergehenden Ansprüche, wobei die zweite Resonanzfrequenz von der ersten Resonanzfrequenz verschieden ist.

6. System (900) nach einem der vorhergehenden Ansprüche, wobei die elektronische Schaltung eine erste Ansteuerschaltungsanordnung, die dazu ausgelegt ist, die erste Unterdruckquelle zu betätigen, und eine zweite Ansteuerschaltungsanordnung, die dazu ausgelegt ist, die zweite Unterdruckquelle zu betätigen, umfasst.

7. System (900) nach einem der vorhergehenden Ansprüche, umfassend:
einen Wundverband, der dazu ausgelegt ist, über einer Wunde eines Patienten platziert zu werden, wobei der Wundverband dazu ausgelegt ist, Fluid zu absorbieren;
wobei die erste Unterdruckquelle (922) auf oder innerhalb des Wundverbandes angeordnet ist,
wobei die zweite Unterdruckquelle (924) auf oder innerhalb des Wundverbandes angeordnet ist,
wobei die elektronische Schaltungsanordnung auf oder innerhalb des Wundverbandes angeordnet ist, wobei die elektronische Schaltungsanordnung dazu ausgelegt ist zu bewirken, dass die erste und die zweite Unterdruckquelle (922, 924) Unterdruck bereitstellen, um Fluid aus der Wunde anzusaugen.

8. System (900) nach Anspruch 7, wobei das Anlegen des ersten Ansteuersignals an den ersten piezoelektrischen Wandler der ersten Unterdruckquelle (922) und das Anlegen des zweiten Ansteuersignals an den zweiten piezoelektrischen Wandler der zweiten Unterdruckquelle (924), die pneumatisch in Reihe geschaltet sind, das Bereitstellen von Unterdruck an die Wunde mit einem maximalen Unterdruckpegel bewirkt, der größer als einzelne maximale Unterdruckpegel der ersten und der zweiten Unterdruckquelle (922, 924) ist.

9. System (900) nach Anspruch 8, wobei der maximale Unterdruckpegel gleich einem kombinierten einzelnen maximalen Unterdruckpegel von erster und zweiter Unterdruckquelle (922, 924) ist.

## Revendications

1. Système de traitement de plaies par pression négative (900) comprenant :
une première source de pression négative (922) configurée pour fournir une pression négative à une plaie recouverte d'un pansement ; et
une seconde source de pression négative (924) configurée pour fournir une pression négative à la plaie couverte par le pansement, la seconde source de pression négative (924) étant reliée pneumatiquement en série à la première source de pression négative (922), la première source de pression négative (922) comprenant un premier transducteur piézoélectrique et la seconde source de pression négative (924) comprenant un second transducteur piézoélectrique, le premier transducteur piézoélectrique ayant une première fréquence de résonance et le second transducteur piézoélectrique ayant une seconde fréquence de résonance ;
**caractérisé en ce que**
le système comprend un circuit électronique configuré pour :
générer un premier signal de commande avec une première amplitude de signal de commande et une première fréquence de signal de commande, la première fréquence de signal de commande correspondant à une moyenne de la première fréquence de résonance et de la seconde fréquence de résonance ;
générer un second signal de commande avec une seconde amplitude de signal de commande et une seconde fréquence de signal de commande, la seconde fréquence de signal de commande correspondant à une moyenne de la première fréquence de résonance et de la seconde fréquence de résonance ; et
appliquer le premier signal de commande au premier transducteur piézoélectrique de la première source de pression négative (922) et appliquer le second signal de commande au second transducteur piézoélectrique de la seconde source de pression négative (924) et amener les première et seconde sources de pression négative (922, 924) à fournir collectivement une pression négative à un niveau de pression négative maximal supérieur aux niveaux de pression négative maximaux individuels des première et seconde sources de pression négative (922, 924).

2. Système (900) selon la revendication 1, comprenant en outre un logement renfermant les première et seconde sources de pression négative (922, 924) et le circuit électronique.

3. Système (900) selon l'une quelconque des revendications précédentes, le niveau de pression négative maximal étant égal à un niveau de pression négative maximal individuel combiné des première et seconde sources de pression négative (922, 924).

4. Système (900) selon l'une quelconque des revendications précédentes, le circuit électronique étant en outre configuré pour déterminer les première et seconde fréquences de résonance à l'initialisation du système, surveiller les première et seconde fréquences de résonance actuelles pendant le fonctionnement du système, et en réponse à une détermination selon laquelle au moins une des première et seconde fréquences de résonance actuelles est différente d'une fréquence de résonance correspondante déterminée lors de l'initialisation du système, régler une fréquence de signal de commande correspondante sur la fréquence de résonance actuelle.

5. Système (900) selon l'une quelconque des revendications précédentes, la seconde fréquence de résonance étant différente de la première fréquence de résonance.

6. Système (900) selon l'une quelconque des revendications précédentes, le circuit électronique comprenant un premier circuit de commande configuré pour actionner la première source de pression négative et un second circuit de commande configuré pour actionner la seconde source de pression négative.

7. Système (900) selon l'une quelconque des revendications précédentes, comprenant :
un pansement configuré pour être placé sur une plaie d'un patient, le pansement étant configuré pour absorber du liquide ;
la première source de pression négative (922) étant disposée sur ou à l'intérieur du pansement,
la seconde source de pression négative (924) étant disposée sur ou à l'intérieur du pansement,
les circuits électroniques étant disposés sur ou à l'intérieur du pansement, les circuits électroniques étant configurés pour amener les première et seconde sources de pression négative (922, 924) à fournir une pression négative pour aspirer le fluide de la plaie.

8. Système (900) selon la revendication 7, l'application du premier signal de commande au premier transducteur piézoélectrique de la première source de pression négative (922) et l'application du second signal de commande au second transducteur piézoélectrique de la seconde source de pression négative (924) relié pneumatiquement en série provoquant la fourniture d'une pression négative à la plaie à un niveau de pression négative maximal qui est supérieur aux niveaux de pression négative maximaux individuels des première et seconde sources de pression négative (922, 924).

9. Système (900) selon la revendication 8, le niveau de pression négative maximal étant égal à un niveau de pression négative maximal individuel combiné des première et seconde sources de pression négative (922, 924).
